# EUROPEAN PATENT APPLICATION

(11) **EP 4 603 477 A1**
(43) Date of publication of application: **20.08.2025**
(21) Application number: 24382161.8
(22) Date of filing: 16.02.2024
(51) Int. Cl.: C07C 233/44, C07C 233/80, C07C 233/15, C07C 235/16, C07C 235/38, A61P 31/14

(54) **INHIBITORS OF THE CORONAVIRUS ACCESSORY PROTEIN ORF9B**

(71) Applicant: Consejo Superior De Investigaciones Científicas, 28006 Madrid (ES); Universitat de Barcelona, 08028 Barcelona (ES)
(72) Inventor: Thomson Okatsu, Timothy, Barcelona (ES); Zodda, Erika, Barcelona (ES); Pons Pons, Mònica, Barcelona (ES); Montoya González, María, Madrid (ES); Díes López-Ayllón, Blanca, Madrid (ES); Calvo González, Cristina, Barcelona (ES); Benítez Rodríguez, Cristina, Barcelona (ES); Rubio Martínez, Jaime, Barcelona (ES); de Moya Valenzuela, Natalia, Barcelona (ES); Cascante Serratosa, Marta, Barcelona (ES); Pujol Dilme, María Dolors, Barcelona (ES); Hibot, Achraf, Barcelona (ES)
(74) Representative: Pons IP

(57) **Abstract**

The invention relates to an inhibitor targeting the coronavirus accessory protein ORF9b of formula **I,** or a pharmaceutical salt thereof, wherein the meanings for the various substituents are as disclosed in the description. Furthermore, the invention relates to its use as a medicament, particularly for the treatment of the hyperinflammation induced by highly pathogenic coronaviruses such as SARS-CoV-2, SARS-CoV or MERS.

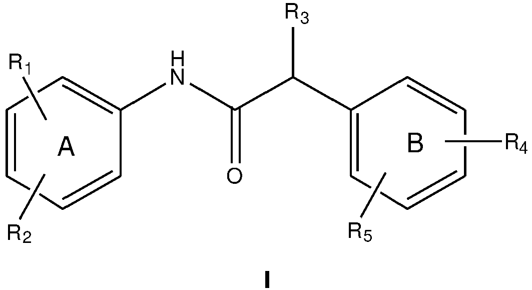

## Description

The invention relates to an inhibitor targeting the coronavirus accessory protein ORF9b of formula **I,** or a pharmaceutical salt thereof. Furthermore, the invention relates to its use as a medicament, particularly for the treatment of the hyperinflammation induced by highly pathogenic coronaviruses such as SARS-CoV-2, SARS-CoV or MERS.

### BACKGROUND ART

Coronaviruses are unique among Nidoviruses in that they encode a variable number of accessory proteins whose functions are not essential for virus replication yet play a relevant role in pathogenesis (Redondo et al., 2021). SARS-CoV-2, the causative agent of COVID-19, is an enveloped virus consisting of a positive-sense, single-stranded RNA genome (Wu et al., 2020a). Two overlapping open-reading frames, ORF1a and ORF1b, are translated from the positive-strand genomic RNA and generate continuous polypeptides, which are cleaved into a total of 16 nonstructural proteins (NSPs). The remaining genomic regions encode four structural proteins: spike (S), envelope (E), membrane (M), and nucleocapsid (N), and six annotated accessory proteins (ORF3a, 6, 7a, 7b, 8, and 10). SARS-CoV-2 also harbors several unannotated accessory ORFs, including alternative open reading frames within ORFs S (ORF2d), N (ORF9b, ORF9c), and ORF3a (ORF3b, ORF3c, ORF3d) (Finkel et al., 2021).

Host-virus interactome analyses have uncovered human proteins that physically associate with SARS-CoV-2 proteins and that may participate in the virus life cycle, infection, replication, and budding (Davies et al., 2020; Gordon et al., 2020a; Gordon et al., 2020b; Chen et al., 2021; Stukalov et al., 2021; Cao et al., 2023). Among these, interactions with mitochondrial proteins are particularly abundant. The interaction of SARS-CoV-2 proteins with mitochondrial proteins results in mitochondrial dysfunction and increased oxidative stress, ultimately leading to the loss of mitochondrial integrity and cell death (Chen et al., 2018; Wu et al., 2020b; Ramachandran et al., 2022). Recent studies suggest that the involvement of mitochondria in SARS-CoV-2 infection is a hallmark of disease pathology (Singh et al., 2020; Holder and Reddy, 2021; Guarnieri et al., 2023) and that it may also explain relevant disease patterns in post-acute COVID syndromes (Chen et al., 2023; Guarnieri et al., 2023).

As a result of compromised mitochondrial integrity, oxidized mitochondrial DNA, cardiolipin and cytochrome c are released into the cytosol, activating damage-associated molecular pattern (DAMP) receptors and Nucleotide-binding oligomerization domain, Leucine rich Repeat and Pyrin domain (NLR)-containing proteins, including NLRP3, with consequent hyper-activation of the inflammasome, leading to sustained local and systemic inflammatory responses (Marchi et al., 2023). Like other viruses, SARS-CoV-2 can activate the inflammasome, thus contributing to unmitigated activation of the innate immune system leading to pathogenicity (Ferreira et al., 2021; Rodrigues et al., 2021; Vora et al., 2021; Junqueira et al., 2022; Sefik et al., 2022; Rodrigues and Zamboni, 2023). Several SARS-CoV-2 structural proteins, including spike (S), nucleocapsid (N), envelope (E) or non-structural proteins, such as nsp6 or ORF3a, have been reported to induce the activation of the inflammasome (Wang et al., 2023). Whether these diverse proteins share common mechanisms to activate the inflammasome is not currently known.

The SARS-CoV-2 accessory protein ORF9b is a small 97 amino acid polypeptide that, in infected cells, forms homodimers that associate with lipids and cell membranes, or monomers that interact with the mitochondrial translocase, TOM70 (Gordon et al., 2020a; Jiang et al., 2020; Gao et al., 2021; Ayinde et al., 2022; Thorne et al., 2022; Jin et al., 2023). The interaction of an ORF9b monomer with TOM70 competes with the binding of the chaperone HSP90 to TOM70, thus leading to its diminished stability and function as a mitochondrial translocase (Brandherm et al., 2021; Gao et al., 2021; Ayinde et al., 2022) and also its ability to interact with MAVS (Thorne et al., 2022) and other critical components of the signaling pathways that induce type I interferons in response to cytosolic viral RNAs (Han et al., 2021; Wu et al., 2021; Thorne et al., 2022). As such, ORF9b is a major factor used by SARS-CoV-2 to interfere with the activation of innate immunity upon viral infection of cells and thus to favor unimpeded viral replication and spread. It follows that inhibition of these ORF9b activities would very likely favor more effective defenses against SARS-CoV-2 infection, lowering viral pathogenesis. Importantly, ORF9b is conserved among SARS-CoV-2, SARS-CoV and other coronaviruses both in sequence and function (Meier et al., 2006; Shi et al., 2014; Gao et al., 2021; Jin et al., 2023). To date, no small-molecule ORF9b inhibitors have been reported.

ORF9b is one of eleven SARS-CoV-2 accessory proteins, not strictly required for viral replication in cell culture, but play essential roles in in vivo viral spread, escape from host immune defenses and pathogenesis. Viral accessory proteins, from coronaviruses, influenza or other viruses with high rates of mutation, are less subject to adaptive evolution and purifying selection than structural proteins. As such, current antivirals become ineffective over time, largely owed to the accrual of adaptive and escape mutations in target viral proteins.

Therefore, it would be desirable to target relevant accessory proteins as a strategy to counter viral pathogenesis less prone to long-term failure than the current strategy of targeting structural proteins. An optimal antiviral strategy would be to target viral accessory proteins in combination with viral structural protein inhibitors. This combinatorial strategy should provide enhanced antiviral efficacies and should be likely less subject to therapeutic failure due to viral target protein adaptive mutations, than current strategies. More specifically, targeting viral factors that afford immune escape and hyperinflammatory responses that accompany viral infection, in combination with current antivirals, would greatly improves the antiviral therapeutic efficacy, and would be less dependent on the emergence of resistant mutants.

### SUMMARY OF THE INVENTION

A first aspect of the present invention relates to a compound of formula I: 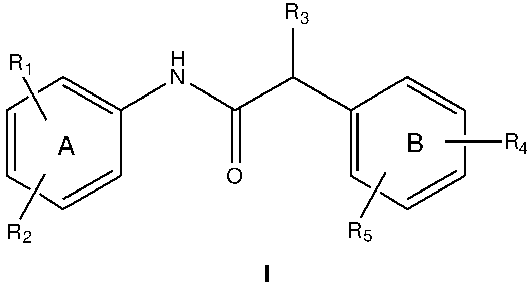 or a pharmaceutical salt thereof, wherein:
R₁ represents -H, -CN, -CF₃, -CHO, -CH₂OH, -F, -Cl, -Br, -I, -NH₂ or -(C₁-C₃)-alkyl;
R₂ represents -NH₂, -NO₂, -NH-OH, -NO, -NH-NH₂, -NH-COCH₃, -NH-COH, -NH-CO- -OEt, -NH-CO-OMe or -NH-CO-NH₂;
R₃ represents -H, -OH or -(C₁-C₃)-alkyl, or, alternatively, R₃ represents a chain selected from -CH=CH-CH=, -CH₂-O-, -CH₂-NH, -CH₂-S, -CH=CH-N=, =CH-O-, =CH-NH- and =CH-S- which is attached to the phenyl ring (B) at C-2 position;
each R₄ and R₅ independently represents -H, -F, -Cl, -Br, -I, -NO₂, -CF₃, -OCH₃, - OCF₃, -SCH₃, -(C₁-C₃)-alkyl, -CN, -COCH₃, -COH, -OH, -NH₂, -COOMe or -COOEt;
phenyl ring (A) is optionally fused to Cy, at any available position;
phenyl ring (B) is optionally fused to Cy₂ at any available position;
each of Cy, and Cy₂ independently represents a 5- or 6-membered carbocyclic ring that can be saturated, partially unsaturated or aromatic, and which can optionally contain 1 or 2 heteroatoms selected from N, S and O, wherein said ring can be bonded to the rest of the molecule through any available C or N atom at any available position, and which can be optionally substituted by 1 or 2 R₆ groups at any available position; and
each R₆ independently represents -H, -F, -Cl, -Br, -I, -NO₂, -CF₃, -OCH₃, -OCF₃, - SCH₃, -(C₁-C₃)-alkyl, -CN, -COCH₃, -COH, -OH, -NH₂, -COOMe or -COOEt.

In another embodiment, the invention relates to a compound of formula **I** as defined above, wherein the compound of formula **I** is a compound of formula **Ia:** 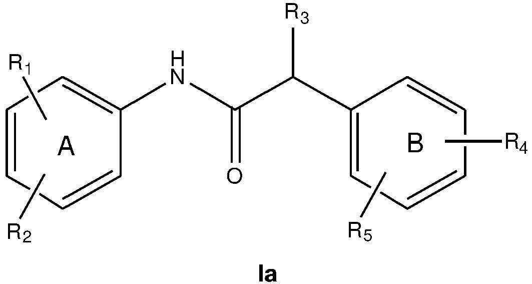 or a pharmaceutical salt thereof, wherein:
R₁ represents -H, -CN, -CF₃, -CHO, -CH₂OH, -F, -Cl, -Br, -I or -(C₁-C₃)-alkyl;
R₂ represents -NH₂, -NO₂, -NH-OH, -NO, -NH-NH₂, -NH-COCH₃, -NH-COH, -NH-CO--OEt, -NH-CO-OMe or -NH-CO-NH₂;
R₃ represents -H, -OH or -(C₁-C₃)-alkyl, or, alternatively, R₃ represents a chain selected from -CH=CH-CH=, -CH₂-O-, -CH₂-NH, -CH₂-S, -CH=CH-N=, =CH-O-, =CH-NH- and =CH-S- which is attached to the phenyl ring (B) at C-2 position; and
each R₄ and R₅ independently represents -H, -F, -Cl, -Br, -I, -NO₂, -CF₃, -OCH₃, - OCF₃, -SCH₃, -(C₁-C₃)-alkyl, -CN, -COCH₃, -COH, -OH, -NH₂, -COOMe or -COOEt.

In another embodiment, the invention relates to a compound of formula **I** as defined above, wherein R₁ represents -H, -CH₃, -F, -Cl, -Br or -I, and, preferably, wherein R₁ represents -CH₃ or -F.

In another embodiment, the invention relates to a compound of formula **I** as defined above, wherein R₂ represents -NH₂ or -NO₂.

In another embodiment, the invention relates to a compound of formula **I** as defined above, wherein R₃ represents -H, -OH or-CH₃, or, alternatively, R₃ represents a CH=CH-CH= chain which is attached to the phenyl ring (B) at C-2 position, and preferably, wherein R₃ represents -H, or, alternatively, R₃ represents a CH=CH-CH= chain which is attached to the phenyl ring (B) at C-2 position.

In another embodiment, the invention relates to a compound of formula **I** as defined above, wherein each R₄ and R₅ independently represents -H, -F, -Cl, -Br, -I or-OCH₃, and, preferably, wherein each R₄ and R₅ independently represents -H, -F, -CI or-OCH₃.

In another embodiment, the invention relates to a compound of formula **I** as defined above, wherein R₄ represents -H.

In another embodiment, the invention relates to a compound of formula **I** as defined above, wherein:
R₄ represents -H; and
R₅ represents -H, -F, -Cl, -Br, -I, -NO₂, -CF₃, -OCH₃, -OCF₃, -SCH₃, -(C₁-C₃)-alkyl, - CN, -COCH₃, -COH, -OH, -NH₂, -COOMe or -COOEt, preferably, R₅ represents -H, - F, -Cl, -Br, -I or -OCH₃, and, more preferably, R₅ represents -H, -F, -CI or -OCH₃.

In another embodiment, the invention relates to a compound of formula **I** as defined above, wherein:
R₁ represents -H, -CH₃, -F, -Cl, -Br or -I, and, preferably, wherein R₁ represents -CH₃ or -F;
R₂ represents -NH₂ or -NO₂;
R₃ represents -H, -OH or-CH₃, or, alternatively, R₃ represents a CH=CH-CH= chain which is attached to the phenyl ring (B) at C-2 position, and, preferably, wherein R₃ represents -H, or, alternatively R₃ represents a CH=CH-CH= chain which is attached to the phenyl ring (B) at C-2 position;
R₄ represents -H; and
R₅ represents -H, -F, -Cl, -Br, -I, -NO₂, -CF₃, -OCH₃, -OCF₃, -SCH₃, -(C₁-C₃)-alkyl, - CN, -COCH₃, -COH, -OH, -NH₂, -COOMe or -COOEt, preferably R₅ represents -H, - F, -Cl, -Br, -I or -OCH₃, and, more preferably, R₅ represents -H, -F, -CI or -OCH₃.

In another embodiment the invention relates to the compound of formula I as defined above, wherein the compound of formula I is selected from:

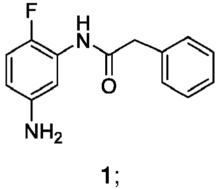

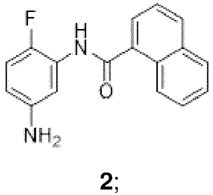

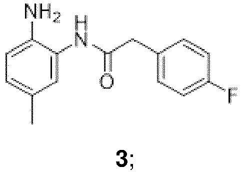

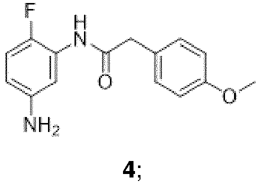

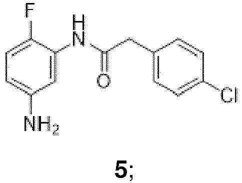

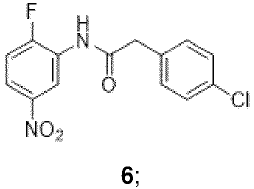

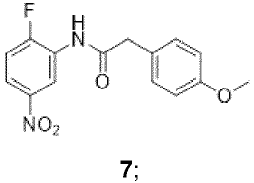

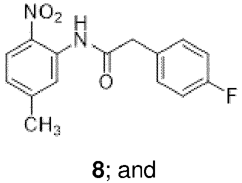

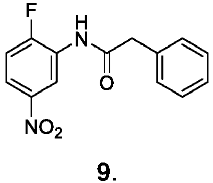

In the above definitions, the term -(C₁-C₃)-alkyl, as a group or part of a group, means a straight or branched alkyl chain which contains from 1 to 3 carbon atoms and include the groups methyl, ethyl, propyl and isopropyl.

A halogen group or its abbreviation halo means fluoro, chloro, bromo or iodo.

The present invention also relates to a pharmaceutical composition that comprises a compound of formula I or a pharmaceutically acceptable salt and a pharmaceutically acceptable excipient. The excipients must be "acceptable" in the sense of being compatible with the other ingredients of the composition and not deleterious to the recipients thereof.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Another aspect of the invention relates to a compound of formula **I** as defined above, or a pharmaceutically acceptable salt thereof, for use as a medicament.

Another aspect of the invention relates to a compound of formula **I** as defined above, or a pharmaceutically acceptable salt thereof, for use in the treatment of hyperinflammation induced by viral infection.

In another embodiment, the invention relates to a compound of formula **I** for the use defined above, wherein viral infection is induced by highly pathogenic coronaviruses, preferably wherein the highly pathogenic coronavirus is selected from SARS-CoV, MERS and SARS-CoV-2, and, more preferably, wherein the highly pathogenic coronavirus is SARS-CoV-2.

Another aspect of the invention relates to the use of a compound of formula **I,** or a pharmaceutically acceptable salt thereof, for the manufacture of a medicament.

Another aspect of the invention relates to the use of a compound of formula **I,** or a pharmaceutically acceptable salt thereof, for the manufacture of a medicament for the treatment of hyperinflammation induced by viral infection, preferably wherein the highly pathogenic coronavirus is selected from SARS-CoV, MERS and SARS-CoV-2, and, more preferably, wherein the highly pathogenic coronavirus is SARS-CoV-2.

Another aspect of the present invention relates to a method of treating a disease in a subject in need thereof, especially a human being, which comprises administering to said subject an effective amount of a compound of formula **I** or a pharmaceutically acceptable salt thereof.

Another aspect of the present invention relates to a method of treating hyperinflammation induced by highly pathogenic coronaviruses, in a subject in need thereof, especially a human being, which comprises administering to said subject an effective amount of a compound of formula **I** or a pharmaceutically acceptable salt thereof, preferably wherein the highly pathogenic coronavirus is selected from SARS-CoV, MERS and SARS-CoV-2, and, more preferably, wherein the highly pathogenic coronavirus is SARS-CoV-2.

Unless otherwise defined, all technical and scientific terms used herein have the same meaning as commonly understood by one ordinary skilled in the art to which this invention belongs. Methods and materials similar or equivalent to those described herein can be used in the practice of the present invention. Throughout the description and claims, the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

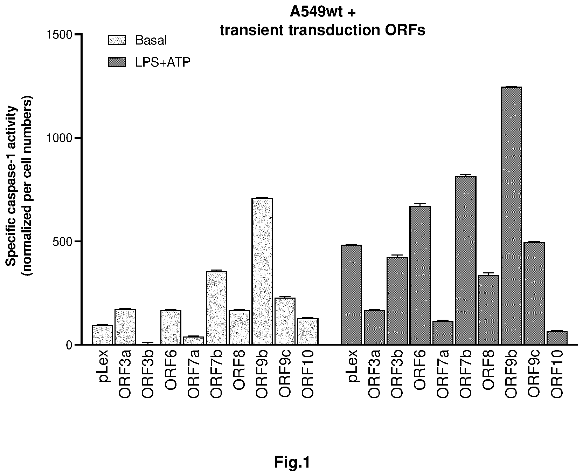

**Figure 1****.** Impact of SARS-CoV-2 accessory proteins on caspase-1 activity. Stable constitutive expression of ORF9b strongly induces caspase-1 activity in A549 lung carcinoma cells. A549 cells stably integrating and constitutively expressing the indicated SARS-CoV-2 accessory proteins were assayed for specific released caspase-1 activity under basal conditions or after exposure to LPS (100 ng/mL) and ATP (5 mM) for 3 h. pLex, A549 integrating the lentiviral vector pLex with no SARS-CoV-2 insert.

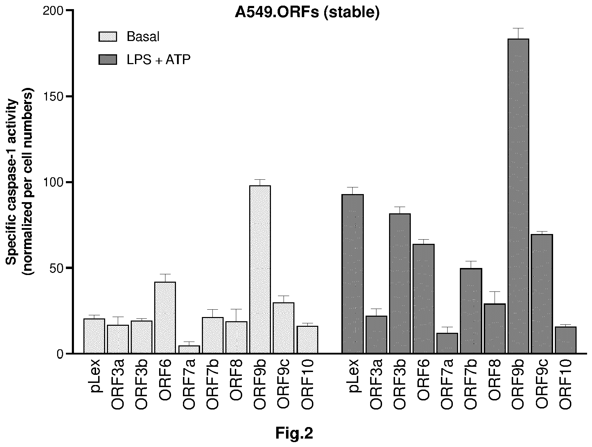

**Figure 2****.** Impact of SARS-CoV-2 accessory proteins on caspase-1 activity. Transient expression of ORF9b strongly induces caspase-1 activity in A549 cells. Parental A549 cells (A549wt) were transduced with lentiviral particles for the expression of the indicated accessory proteins and released caspase-1 activity determined 48 h after transduction, under basal conditions or after exposure to LPS+ATP for 3 h. pLex, transduction of lentiviral particles bearing the control vector pLex.

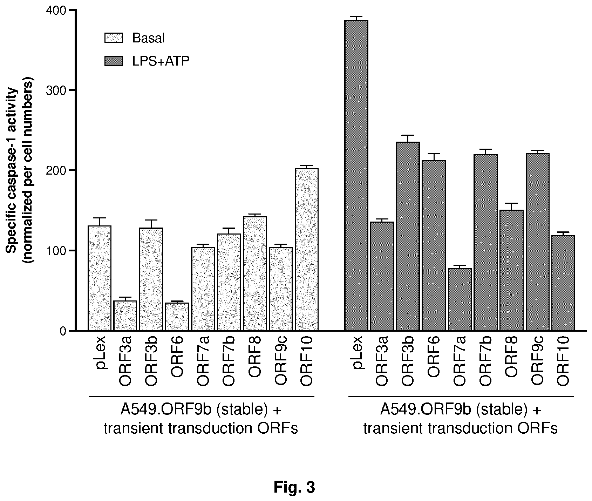

**Figure 3****.** Impact of SARS-CoV-2 accessory proteins on caspase-1 activity. A549 cells with stable integration and expression of ORF9b (A549.ORF9b) were transduced with lentiviruses for the expression of the indicated ORFs and released caspase-1 activity determined 48 h after transduction, under basal conditions or after exposure to LPS+ATP for 3h. pLex, transduction of lentiviral particles bearing the control vector pLex.

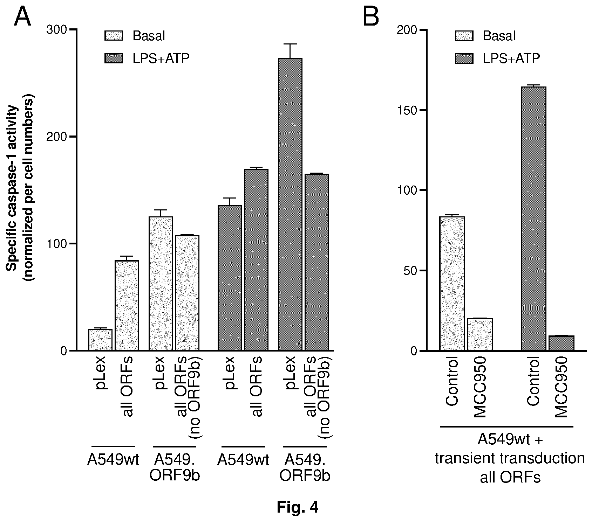

**Figure 4****.** Impact of SARS-CoV-2 accessory proteins on caspase-1 activity. **A.** The induction of caspase-1 activity by ORF9b is dominant among all accessory proteins when concomitantly expressed in A549wt or A549.ORF9b cells. The concomitant expression of all accessory proteins abrogates the LPS+ATP-induced additive induction of caspase-1 activity to that induced by ORF9b alone. Parental A549wt or A549.ORF9b cells were transduced with equal titers of lentiviral particles for each accessory protein and assayed for released caspase-1 activity after 48 h. pLex, transduction of lentiviral particles bearing the control vector pLex. **B.** The induction of caspase-1 activity by the concomitant expression of all SARS-CoV-2 accessory proteins in A549wt cells, without or with additional boosting by LPS+ATP, is dependent on the NLRP3 inflammasome. Parental A549wt cells were transduced with equal titers of lentiviral particles for each accessory protein and assayed for released caspase-1 activity after 48 h. Cells were treated with the NLRP3 inflammasome inhibitor MCC950 for the last 3 h or vehicle (control).

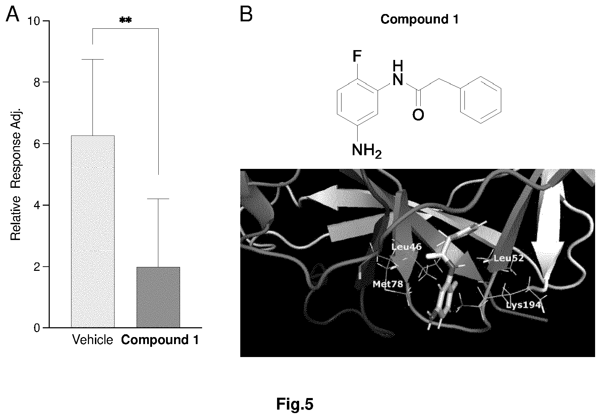

**Figure 5****.** A small compound that is an effective inhibitor of ORF9b-ORF9b homotypic interaction. **A.** Surface plasmon resonance (SPR) determination of ORF9b homodimerization surface-binding hit compound Compound 1 on ORF9b-ORF9b interactions. Recombinant GST-ORF9b protein was immobilized on CM5 chips and interactions with mobile-phase GST-ORF9b compounds recorded by SPR, in the absence (vehicle) or presence of the indicated compound at 5 µM final concentration. Determinations were performed in triplicate. **B.** Structure and ribbon model of the Compound 1 in inhibition of ORF9b homodimerization interaction. For the ribbon rendering, the most relevant residues for on ORF9b for its interaction with each compound are illustrated, all located on the homodimerization surface.

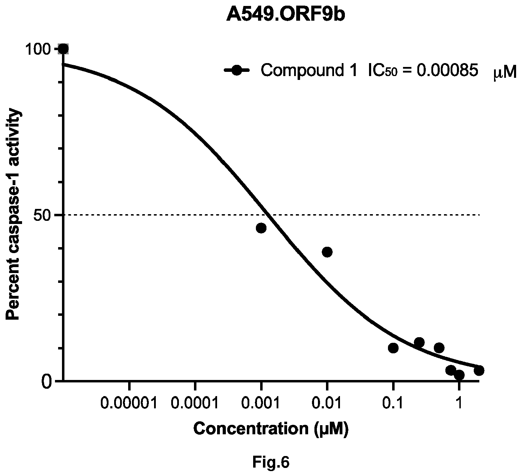

**Figure 6****.** ORF9b homodimerization inhibitor Compound 1 inhibits the activation of caspase-1 induced by ORF9b in A549 lung epithelial cells. A549 cells stably integrating and constitutively expressing ORF9b (A549.ORF9b cells) were either incubated with 5 µM of each compound (or solvent) for 3 h, and their cell culture supernatants collected for caspase-1 activity determination

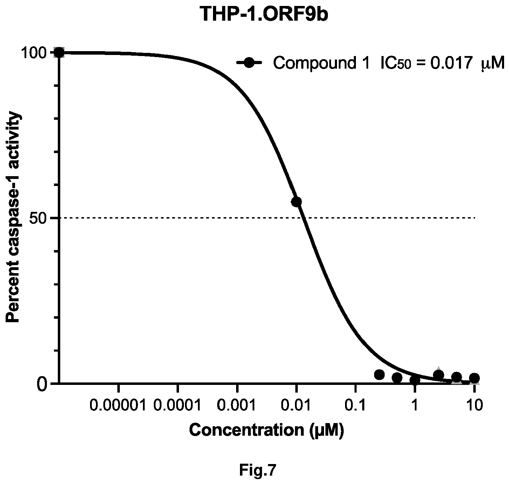

**Figure 7****.** ORF9b homodimerization inhibitor Compound 1 inhibits the activation of caspase-1 induced by ORF9b in THP-1 monocyte-macrophage cells. THP-1 cells stably integrating and constitutively expressing ORF9b (THP-1.ORF9b cells) were either incubated with 5 µM of each compound (or solvent) for 3 h, and their cell culture supernatants collected for caspase-1 activity determination.

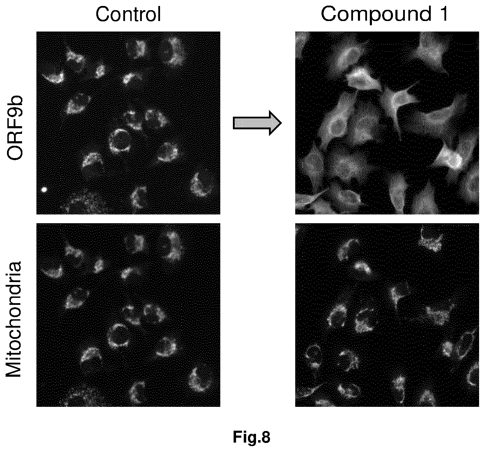

**Figure 8****.** Compound 1 causes mitochondrial eviction of ORF9b in A549.ORF9b cells. Cells were incubated with 2.5 µM of Compound 1 (or solvent) for 4 h. Mitochondria were visualized by incorporation of MitoView Fix 640 (green channel). After incubations, cells were fixed and permeabilized and ORF9b detected by immunofluorescence with a specific rabbit anti-ORF9b antibody and a secondary antibody conjugated with Alexa 568 (red channel).

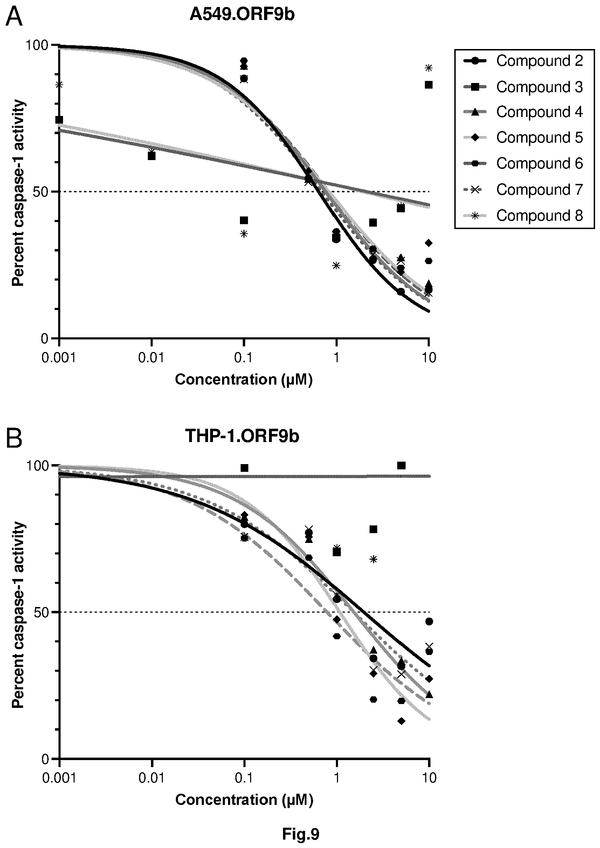

**Figure 9****.** Compound 1 derivative compounds inhibit ORF9b-mediated activities. **A.** Compounds 2, 4, 5, 6 and 7, but not Compounds 3 and 8, inhibit the activation of caspase-1 induced by ORF9b in A549 lung epithelial cells at low micromolar concentrations. **B.** Compound 1 derivative compounds inhibit ORF9b-mediated activities. Compounds 2, 4, 5, 6 and 7, but not Compounds 3 and 8, inhibit the activation of caspase-1 induced by ORF9b in THP-1 monocyte-macrophage cells at low micromolar concentrations.

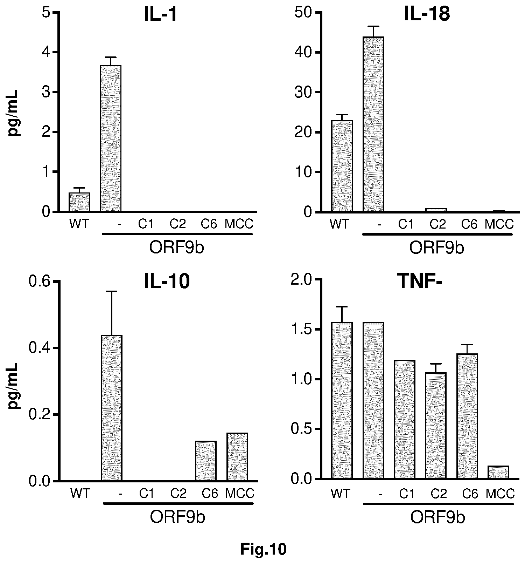

**Figure 10****.** Compound 1-derivative compounds inhibit ORF9b-mediated activities. Compounds 1, 2 and 6 inhibit the secretion of the indicated cytokines induced by ORF9b in THP-1 monocyte-macrophage cells. The NLRP3 inflammasome inhibitor, MCC950 (MCC) was used as a control inhibitor.

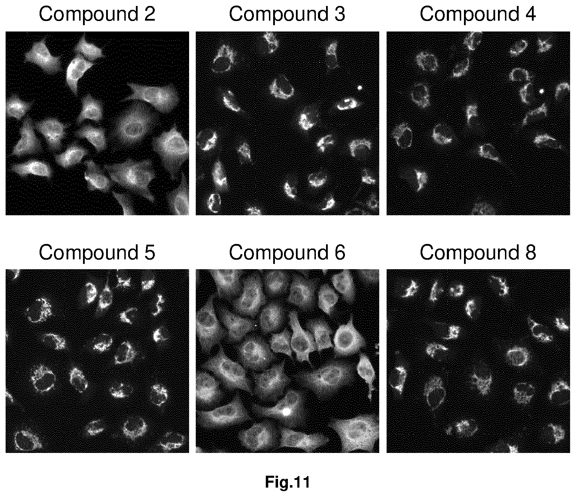

**Figure 11****.** Compounds 2 and 6, but not Compounds 3, 4, 5 or 8, induce mitochondrial eviction of ORF9b in A549.ORF9b cells. Cells were incubated with 5 µM of each compound (or solvent) for 4 h. After incubations, cells were fixed and permeabilized and ORF9b detected by immunofluorescence with a specific rabbit anti-ORF9b antibody and a secondary antibody conjugated with Alexa 568 (red channel).

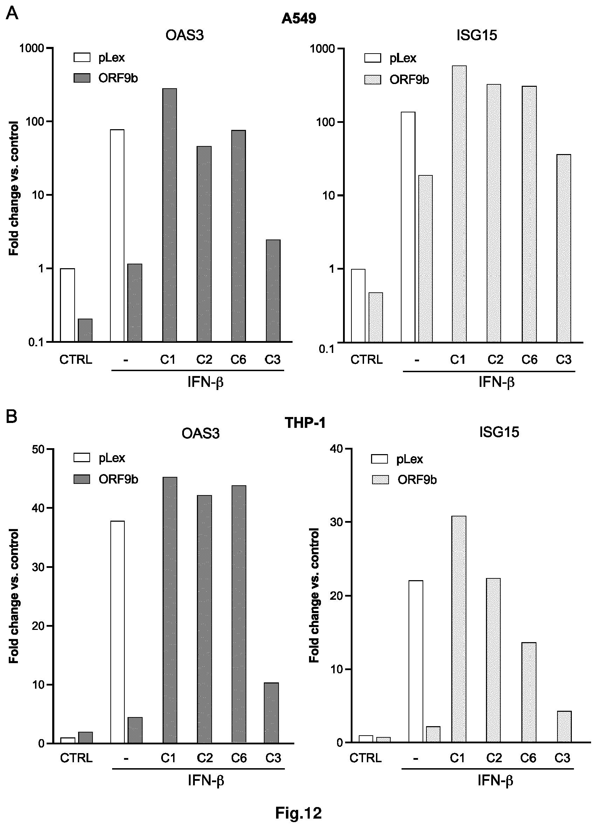

**Figure 12****.** Compound 1 and derivative compounds restore type-I interferon responses compromised by ORF9b. **A.** ORF9b inhibits the induction by IFN-β of OAS3 and ISG15 in A549 cells. A549 cells were transduced with a lentiviral vector for the expression of ORF9b, or with a control empty lentiviral vector (pLex) and, 24 later, exposed to 10 ng/mL of recombinant human interferon β1 (IFNβ) for 4 h, and RNA isolated and transcript levels quantitated by real-time RT-PCR. **B.** ORF9b inhibits the induction by IFN-β of OAS3 and ISG15 in THP-1 cells. THP-1 cells were transduced with a lentiviral vector for the expression of ORF9b, or with a control empty lentiviral vector (pLex) and, 24 later, were exposed to 10 ng/mL of recombinant human interferon β1 (IFNβ) for 4 h, and RNA isolated and transcript levels quantitated by real-time RT-PCR.

### EXAMPLES

### Virtual screening for ORF9b homodimerization inhibitors

The atomic coordinates of the solved structure of the SARS-CoV-2 ORF9b homodimer (PDB: 6Z4U) were used as the initial scaffold for virtual screening. Multiple classical and accelerated molecular dynamics (GaMD) simulations (Rubio-Martinez et al., 2021) were performed at different initial temperatures. This temperature change ensures that each molecular dynamics starts with a different velocity distribution, which can have a significant impact on the behavior and properties during the simulation and, thus, explore different states and trajectories of the system. A direct docking process was conducted with the AutoDock Vina software (Trott and Olson, 2010; Eberhardt et al., 2021), using as receptors structures selected from the GaMD simulations, and as ligands compounds from the Fragment Library and Diversity Library from the European Chemical Biology Library (https://www.eu-openscreen.eu/services/compound-collection.html). Ligand-receptor complexes were selected based on energy thresholds, the systems minimized, and free binding energy calculated using the MMPBSA method. Structures present in the largest number of representative receptor structures were chosen, and molecular dynamics simulations performed. The systems were selected through an iterative process of molecular dynamics calculations, energy rescoring and ligand selection. A total of 500 ns with cMD and 400 ns with GaMD were simulated. This process was replicated for both the dimer and the monomer of ORF9b.

### Compound synthesis

Compounds of formula **I** may be synthesized by using some of the methods described below, as well as other processes known in the field of the organic chemistry. Preferred methods include, but are not limited to, the general procedures shown in the following Schemes.

### General methods.

Melting points were obtained in open capillary tubes on an MFB-595010M Gallenkamp apparatus equipped with a digital thermometer. IR spectra were obtained using a FTIR Perkin-Elmer 1600 Infrared Spectrophotometer. Only noteworthy IR absorptions are listed (cm⁻¹). ¹H and ¹³C NMR spectra were recorded at room temperature on a Brucker-400 (400 and 100.6 MHz respectively) or Varian Gemini-400 (400 and 100.6 MHz) instruments using CDCl₃ as solvent with tetramethylsilane as internal standard, (CD3)2CO or (CD3)2SO. Other ¹H, ¹³C NMR spectra and heterocorrelation 1H-¹³C (HSQC and HMBC) experiments were recorded on a Varian Gemini-400 (400 MHz and 100.6 MHz) instrument. Chemical shifts (δ scale) are reported in parts per million (ppm) relative to the central peak of the solvent (δ = 7.26 ppm for CDCl₃ in ¹H NMR and δ = 77.16 ppm for CDCl₃ in ¹³C NMR). Mass spectra were taken on a Hewlett-Packard 5988-A instrument and high-resolution mass spectra (HRMS) were recorded on a LC/MSD-TOF mass spectrometer (Agilent Technologies). Column chromatography was performed with silica gel (E. Merck, 70-230 mesh). Reactions were monitored by TLC using 0.25 mm F-254 silica gel (E. Merck). Elemental analysis for C, H and N were determined on a Carlo Erba-1106 analyzer. All reagents were of commercially quality or were purified before use.

Organic solvents were of analytical grade or were purified by standard procedures. Reactions were carried out under argon.

**Method for nitro-amide synthesis. Method A-1.** To a solution of the appropriate aniline (1 eq) in 10 mL of dichloromethane, trimethylamine (1 eq) was added to a solution of the appropriate aniline (1 eq) in 10 mL dichloromethane. Then the appropriate carboxylic acid (1 eq) and thionyl chloride were added slowly to the reaction, and the reaction mixture stirred at room temperature for 24 hours. Finally, the solvent was evaporated under reduced pressure and the product recrystallized with a mixture of ethyl acetate and hexane.

**Method for nitro-amide synthesis. Method A-2.** The corresponding acid chloride (1.1 eq) was added to a solution of the nitroaniline (1 eq) in dichloromethane (20 mL) previously cooled to 0° C with an external ice bath. After addition, the reaction mixture was kept under stirring for 6 h. Reaction controls were carried out by TLC. Once the starting product had been exhausted, the dichloromethane phase was washed with a 1N NaOH solution (3 x 20 mL) and the organic phase was dried over Na₂SO₄. Next, the organic phase was filtered and the solvent evaporated under reduced pressure. The obtained crude product was purified by column chromatography using hexane and ethyl acetate as eluents or crystallized from mixtures of hexane and ethyl acetate.

**Method for nitro-amide synthesis. Method A-3.** TiCl₄ (0.738 g, 0.42 mL, 3.891 mmol) and the 5-methyl-2-nitroaniline (0.197 g, 1.297 mmol) were added to a solution of 4-fluorophenylacetic acid (0.2g, 1.297mmol) in pyridine (10 mL). The tightly sealed screw-capped vial containing the reaction mixture was then heated at 120° C. After magnetic stirring for about 72 h, TLC analysis (AcOEt/Hex 20:80 v/v) of the reaction mixture showed complete conversion of the carboxylic acid precursor. The reaction mixture was then cooled, neutralized with HCl solution (2N). The green precipitate obtained was filtered, washed several times with distilled water and dried at 50 °C.

**Method for reduction of nitro-amides. Method B.** The corresponding nitro-amide (1 eq) was treated with iron powder (10 eq) in acetic acid (10 mL) at reflux for 24 hours. At the end of the TLC-controlled reaction, the crude mixture was filtered under reduced pressure followed by neutralization with a 5% ammonia solution. Ethyl acetate (3 x 20 mL) was used to extract the crude reaction product, the organic phases combined, dried over anhydrous Na₂SO₄ and filtered under reduced obtain the product. The aminoamide obtained was purified by column chromatography or by recrystallization with hexane/ethyl acetate/hexane mixtures.

### N-(5-Amino)-2-fluorophenyl)-2-phenylacetamide (Compound 1)

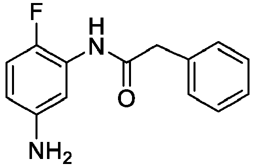

Starting from 200 mg (1.28 mmol) of the 2-fluoro-5-nitroaniline and following the procedure **A-2** was obtained the corresponding amide as a white solid in acceptable yield. Log P = 2.85. The nitro amide obtained, was reduced following the procedure **B.** After purification by chromatography column (hexane-ethyl acetate 8:2) was obtained the aminoamide **1** in 51% yield (136.94 mg, 0.56 mmol). Lop P = 2.14. Aspect white solid.

### N-(5-Amino-2-fluorophenyl)-1-naphthamide (Compound 2)

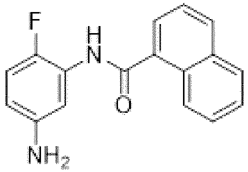

Starting from 200 mg (1.28 mmol) of the 2-fluoro-5-nitroaniline and following the procedure **A-2** was obtained the corresponding amide as a yellow solid in a 67% of yield. Log P = 3.79. NMR ¹H (CDCl₃, 400 MHz) δ (ppm): 7.30 (t, *J=* 9.2 Hz, 1H, H-7); 7.53-7.60 (m, 2H, H-4, H-6); 7.80 (d, *J=* 7 Hz, 1H, H-3); 7.92 (d, *J=* 7Hz, 1H, H-5); 8.02-8-08 (m, 3H, H-2, H-3', H-6'); 8.40 (d, *J* = 8 Hz, 1H, H-4'); 9.52 (dd, *J* = 2.7, *J* = 9.2 Hz, 1H, H-8). The nitro amide obtained previously 390 mg (1.25 mmol) was reduced following the procedure **B.** After purification by chromatography column (hexane-ethyl acetate 7:3) was obtained the aminoamide 2 in 46% yield. Log P = 3.19. Aspect: brown solid. Mp: 151-153 °C (ethyl acetate) NMR ¹H (CDCl₃, 400 MHz) δ (ppm): 6.37-6.39 (m, 1H, H-4'); 6.91 (t, *J* = 8.7 Hz, 1H, H-6); 7.50-7.59 (m, 3H, H-3, H-3', H-6'); 7.74 (d, *J* = 7Hz, 1H, H-5); 7.89-7.91 (m, 2H, H-4, H-7); 7.97 (d, *J=* 8 Hz, 1H, H-2); 8.00 (bs, 1H, NH); 8.40 (dd, *J* = 1.0, *J* = 8.0 Hz, 1H, H-8).

### N-(2-Amino-5-methylphenyl)-2-(4-fluorophenyl)acetamide (Compound 3)

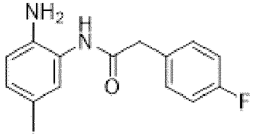

Starting from 130 mg (0.45 mmol) of the corresponding nitroamide **8** and following the general procedure **B** was obtained the desired aminoamide **3** in 36% of yield (43.0 mg). Log P = 2.63. Aspect: brown semi-solid. NMR ¹H (CDCl₃, 400 MHz) δ (ppm): 2.37 (s, 3H, CH₃-); 4.08 (s, 2H, CH₂-Ar); 6.80 (t, *J* = 6.7 Hz, 2H, H-3, H-5); 7.01 (dd, *J* = 1.5, *J* = 8.2 Hz, 1H, H-4'); 7.09 (dd, *J* = 1, *J* = 6.7 Hz, 2H, H-2, H-6); 7.21 (ba, 1H, H-6'); 7.34 (d, *J* = 8.2 Hz, 1H, H-3').

### N-(5-Amino-2-fluorophenyl)-2-(4-methoxyphenyl)acetamide (Compound 4)

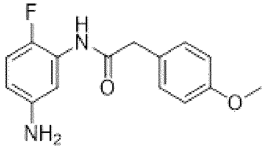

Starting from 100 mg (0.329 mmol) of the nitroamide **7** and following the general procedure **B** was obtained the desired aminoamide **4** in 39% of yield (35.2 mg). Log P = 2.01. Aspect: brown solid. Mp: 147-149 °C NMR ¹H (CDCl₃, 400 MHz) δ (ppm): 3.83 (s, 2H, CH₂-Ar); 3.91 (s, 3H, CH₂-O); 4.94 (bs, 2H, NH₂); 6.95-7.02 (m, 4H, H-3, H-5, H-4', H-6'); 7.26-7.28 (m, 2H, H-2, H-6); 7.87-7.89 (m, 1H, H-4'); 8.02 (d, *J=* 2.8 Hz, 1H, H-6'); 9.9 (bs, 1H, NH).

### N-(5-Amino-2-fluorophenyl)-2-(4-chlorophenyl)acetamide (Compound 5)

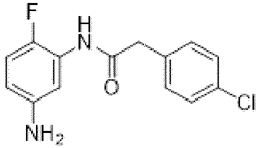

Starting from 100 mg (0.323 mmol) of the corresponding nitroamide **6** and following the general procedure **B** was obtained the desired aminoamide **5** in 42% of yield (37.7 mg). Log P = 2.70. Aspect: brown semi-solid. NMR ¹H (CDCl₃, 400 MHz) δ (ppm): 3.73 (s, 2H, CH₂-Ar); 6.98 (t, *J* = 9.2 Hz, 1H, H-3'); 7.27 (d, *J* = 6 Hz, 2H, H-3, H-5); 7.35 (d, *J=* 7 Hz, 2H, H-2, H-6); 7.47 (bs, 2H, NH₂); 7.43 (bs, 1H, NH-amide); 7.60-7.63 (m, 1H, H-4'); 8.02 (d, *J* = 2.8 Hz, 1H, H-6').

### 2-(4-Chlorophenyl)-N-(2-fluoro-5-nitrophenyl)acetamide (Compound 6)

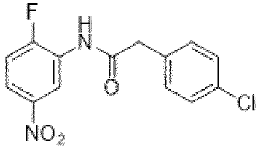

Starting from 183 mg (1.172 mmol) of 2-fluoro-5-nitroaniline and following the general procedure **A-1** was obtained the desired nitroamide **6** in 79% of yield (420 mg). Log P = 3.47. Aspect: white solid. Mp: 224-226 °C. NMR ¹H (CDCl₃, 400 MHz) δ (ppm): 3.78 (s, 2H, CH₂-Ar); 7.19 (t, *J* = 9.0 Hz, 1H, H-3'); 7.29 (d, *J* = 7 Hz, 2H, H-3, H-5); 7.40 (d, *J* = 7 Hz, 2H, H-2, H-6); 7.43 (bs, 1H, NH-amide); 7.92-7.96 (m, 1H, H-4'); 9.02 (d, *J=* 2.8 Hz, 1H, H-6').

### N-(2-Fluoro-5-nitrophenyl)-2-(4-methoxyphenyl)acetamide (Compound 7)

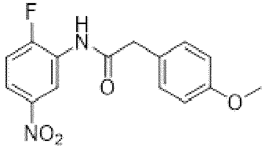

Starting from 470 mg (3.0 mmol) of 2-fluoro-5-nitroaniline and following the general procedure **A-1** was obtained the desired nitroamide **7** in 52% of yield (420 mg). Log P = 2.98. Aspect: white solid. Mp: 181-183 °C. NMR ¹H (CDCl₃, 400 MHz) δ (ppm): 3.76 (s, 2H, CH₂-Ar); 3.78 (s, 3H, CH₃-O); 6.92 (d, *J* = 6.6 Hz, 2H, H-3, H-5); 7.15 (t, *J=* 9.0 Hz, 1H, H-3'); 7.25 (d, *J=* 7 Hz, 2H, H-2, H-6); 7.60 (bs, 1H, NH-amide); 7.92-7.96 (m, 1H, H-4'); 9.26 (d, *J* = 2.8 Hz, 1H, H-6').

### N-(2-Nitro-5-methylphenyl)-2-(4-fluorophenyl)acetamide (Compound 8)

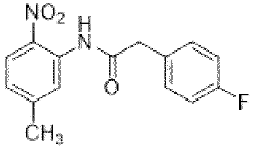

Starting from 197 mg (1.3 mmol) of 2-nitro-5-methylaniline and following the general procedure **A-3** was obtained the desired nitroamide **8** in 98% of yield (367 mg). Log P = 3.27. Aspect: white solid. Mp: 257-259 °C. NMR ¹H (CDCl₃, 400 MHz) δ (ppm): 3.78 (s, 2H, CH₂-Ar); 6.01 (bs, 1H, NH); 6.94 (dd, *J* = 1, *J* = 8.6 Hz, 1H, H-4'); 7.11 (t, *J=* 5.2 Hz, 2H, H-3, H-5); 7.13 (dd, *J* = 3.6, *J* = 5.2 Hz, 2H, H-2, H-6); 8.00 (d, *J=* 8.6 Hz, 1H, H-3'); 8.60 (d, *J=* 1 Hz, 1H, H-6').

### N-(2-Fluoro-5-nitrophenyl)-2-phenylacetamide (Compound 9)

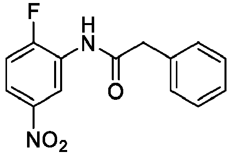

Starting from 200 mg (1.28 mmol) of the 2-fluoro-5-nitroaniline and following the procedure **A-2** was obtained the corresponding amide. After purification by chromatography column (hexane-ethyl acetate 8:2) and followed by recrystallization from a 5:5 ethyl acetate:hexane the aminoamide **9** in 91% yield (283.66 mg, 1.16 mmol) was obtained. Lop P = 2.85. Aspect: white solid. Mp: 156-157 °C. NMR ¹H (CDCl₃, 400 MHz) δ(ppm): 3.86 (s, 2H, CH2-Ar); 7.18 (t, *J=* 9.0 Hz, 1H, H-4 (phenyl)); 7.36-7.43 (m, 3H, H-3, H-5 (phenyl) H-3 (fluoronitrophenyl)); 7.43 (d, *J=* 9 Hz, 2H, H-2, H-6 (phenyl)); 7.67 (bs, 1H, NH-amide); 7.95-7.98 (m, 1H, H-4 (fluoronitrophenyl); 9.27 (dd, *J* = 2.8 Hz, *J* = 6.8Hz, 1H, H-6 (fluoronitrophenyl).

### Biochemical and biological activity (1). Surface plasmon resonance.

Recombinant SARS-CoV-2 GST-ORF9b protein (BPS Bioscience) was immobilized on a Sensor Chip CM5 channel (Cytiva). Control GST protein was immobilized on a second channel of the same chip. For the mobile phase, GST-ORF9b was maintained at 400 nM in running buffer (10 mM HEPES, 0.15 M NaCl, pH 7.4). To assess its activity on the homotypic interaction with solid-phase GST-ORF9b, mobile-phase GST-ORF9b was preincubated with the test compounds at varying concentrations, and interaction assays run on a Biacore T100 instrument. These determinations were performed at least in triplicate. Reductions ≥ 60% in homotypic binding of GST-ORF9b were considered an inhibitory response.

### Biochemical and biological activity (2). Caspase-1 activity.

A549 pulmonary epithelial cells and THP-1 cells were cultured in Dulbecco's Modified Eagle Medium (DMEM) (Gibco) supplemented with 10% (v/v) heat-inactivated fetal bovine serum (FBS) (Gibco), 1% Penicillin-Streptomycin (100 U/ml) (Gibco) and Amphotericin B (Gibco). SARS-CoV-2 (Wuhan-Hu-1 isolate) accessory proteins coding sequences (codon-optimized for mammalian expression) were cloned into pLVX-EF1α-IRES-Puro Cloning and Expression Lentivector (Clontech) to generate pseudotyped lentiviral particles. Accessory proteins were C-terminally 2 x Strep-tagged to check viral protein expression. Vectors were generated and used for the expression of the following SARS-CoV-2 accessory proteins: ORF3a, ORF3b, ORF6, ORF7a, ORF7b, ORF8, ORF9b, ORF9 and ORF10. Control vectors were either pVLX-EF1A-IRES-Puro or pLEX, with no insert. For transient experiments, transduction was performed at a multiplicity of infection (Mol) of ≈ 10 to ensure expression of the transduced viral proteins in all cells, followed by experimental perturbations 48 h after transduction. For stable Integration of SARS-CoV-2 accessory proteins, A549 cells were exposed to puromycin (2 µg/mL) 24 h after transduction and selected for 3 d. All cells were cultured at 37 °C in a 5% CO₂, 90% humidity atmosphere.

Released caspase-1 enzymatic activity of was quantitated with the Caspase-Glo 1 inflammasome assay (Promega), following instructions from the supplier. Briefly, cells were seeded, subjected to experimental perturbations, and 50 µL of cell culture supernatant was added to a mixture containing the selective caspase-1 substrate, Z-WEHD-aminoluciferin, and MG-132 protease inhibitor, with or without the irreversible caspase-1 inhibitor, Z-YVAD-FMK (Sigma-Aldrich), and luminescence measured on a Cytation 5 instrument (Agilent). The luminescence inhibited by Z-YVAD-FMK corresponds to specific caspase-1 activity. To ascertain the effect of ORF9b homodimerization inhibitor compounds on ORF9b-dependent caspase-1 activation, dose-response determinations were performed by exposing A549 or THP-1 cells, stably or transiently expressing ORF9b, to varying concentrations of each compound in the cell culture medium for 24 h, and 50% inhibitory concentrations (IC50) of the resulting caspase-1 activity were calculated. All assays were done in triplicate.

### Biochemical and biological activity (3). Cytokine quantification.

THP-1 cells stably integrating and constitutively expressing ORF9b were generated as described under "Biochemical and biological activity (2)". Cytokines (IL-1β, IL-18, IL-10, TNF-α) were quantified by means of Procartaplex multiplex assays (InVitrogen, ThermoFisher), following instructions by the supplier. Briefly, capture beads were incubated for 2 h with cleared cell supernatants, washed and incubated with biotinylated detection antibody mix, incubated for 30 min, washed and incubated with Streptavidin-PE for 30 min, washed and data acquired on a Luminex 200 instrument. To ascertain the effect of ORF9b homodimerization inhibitors on ORF9b-mediated cytokine secretion, THP1-1 cells with the above modifications were treated with 1 µM or 5 µM of each of the compounds (final concentrations in cell culture media), and cytokine levels determined in cell culture supernatants. All determinations were performed in triplicate, and analyzed statistically significance in differences by means of a two-tailed Student's *t-*test. Inhibition of ORF9b-mediated secretion of cytokines was considered significant with *p* values ≤ 0.05.

### Biochemical and biological activity (4). Fluorescence microscopy.

A549 cells stably integrating and constitutively expressing SARS-CoV-2 ORF3a, ORF3b, ORF6, ORF7a, ORF7b, ORF8, ORF9b, ORF9C or ORF10, were generated as described under "Biochemical and biological activity (2)". Cells were seeded on 96-well flat bottom microplates (Greiner 655090) and allowed to attach for 24 h, and incubated for 3 h 45 min with 2.5 to 5 µM of compounds EN300 (Compound 1), Y600, Y203 or Compounds 2 through 8 (or solvent) and 100 nM Mitoview Fix 640 (Biotium) for mitochondrial visualization. After these incubations, cells were washed with PBS 1x, fixed with 4% paraformaldehyde/PBS during 15 min and, after washing with PBS 1x, blocked for 30 min at room temperature with 1% BSA/PBS and 0.1% Triton X-100, followed by incubation with primary antibody (rabbit polyclonal anti-SARS-CoV-2 ORF9b, Abyntek #9191) diluted in blocking buffer during 1 h at room temperature. Subsequently, after washing with PBS 1x, cells were incubated with secondary antibody (AlexaFluor-568-conjugated donkey anti-rabbit IgG(H+L), Invitrogen) 1:2,000 in blocking buffer containing also 4,6-diamidino-2-phenylindole (DAPI, 1/10,000 dilution), for 1 h at room temperature on a shaker. Fluorescence excitation was done at 490 nm and 568 nm wavelengths, and images captured at 523 nm (green channel, mitochondrial) and 603 nm (red channel, ORF9b), respectively. All images were captured with a Leica Stellaris 8 Confocal microscope. Co-localization of ORF9b and mitochondria was assessed visually.

### Biochemical and biological activity (5). Real-time quantitative RT-PCR.

A549 and THP-1 cells stably integrating and constitutively expressing SARS-CoV-2 ORF9b were generated as described under "Biochemical and biological activity (2)". Cells were either treated with recombinant human 10 ng/mL interferon-β1 (Prprotec) for 4 h, and further treated with ORF9b homodimerization inhibitors at 1 µM final concentrations (or diluted DMSO, as control) for 1 h. After treatment, cells were rinsed with PBS and plates frozen. RNA was isolated using Trizol reagent (Invitrogen). Reverse transcription was carried out with 1 µg RNA at 37 °C for 1 h with the following reagents: 5x reverse transcription buffer (Invitrogen), 0.1 M dithiothreitol (DTT) (Invitrogen), Random Hexamers (Roche), 40 U/µL RNAsin (Promega, Fitchburg, Wl, USA), 40 mM dNTPs (Bioline, London, UK), 200 U/µL M-MLV-RT (Invitrogen). Gene expression analysis was performed on an Applied Biosystems Quantstudio 3 Real-Time PCR System, using TaqMan gene specific sequences for OAS3 (Hs00196324_m1) or ISG15 (Hs01921425_s1) (Applied Biosystems, Thermo Fisher Scientific Inc.). Reactions were performed in a final volume of 20 µL, containing 9 µL of cDNA mixture and 11 µL of the specific TaqMan assay in reaction Master Mix (Applied Biosystems). Real-Time PCR was conducted according to the following parameters: an initial incubation at 50 ºC for 2 min and denaturalization at 95 ºC for 10 min, followed by 40 cycles at 95 ºC and 60 ºC for 15 sec and 1 min, respectively. Expression was quantified by the ΔΔCt method using Rna18s5 (Hs03928985_g1, Applied Biosystems) as a reference transcript. All determinations were done in triplicate. Fold-change expression levels were compared between control cells (without ORF9b) *vs.* cells expressing ORF9b, untreated vs. treated with IFNB1, and treated with ORF9b dimerization inhibitors vs. control. Fold-change values were analyzed for statistically significance in differences by means of a two-tailed Student's t-test. Inhibition by ORF9b dimerization inhibitors of ORF9b-mediated suppression of INFβ1-induced gene expression was considered significant with *p* values ≤ 0.05.

### Results

### ORF9b induces caspase-1 activation uniquely among SARS-CoV-2 accessory proteins.

In order to determine the ability of SARS-CoV-2 accessory proteins to modulate the activation of the inflammasome, caspase-1 activity was assessed, as a proxy of inflammasome activation, of A549 lung carcinoma cells either stably integrating, or transiently transduced with, ORF3a, ORF3b, ORF6, ORF7a, ORF7b, ORF8, ORF9b, ORF9c or ORF10 under basal conditions or after exposure to the toll-lie receptor 4 (TLR4) agonist, lipopolysaccharide (LPS) followed by extracellular ATP (LPS+ATP). Both stable, long-term constitutive expression of SARS-CoV-2 accessory proteins and transient transduction and expression of the same ORFs, impacted caspase-1 activation to similar degrees, perhaps with the exception of ORF7b (Fig. 1 and 2). Uniquely, expression of ORF9b strongly induced caspase-1 activity under both experimental conditions (Fig. 1 and 2). On the other hand, both long-term and transient expression of ORF7a, but only transient expression of ORF3d, suppressed basal caspase-1 activity (Fig. 2). As expected, exposure to LPS+ATP also strongly induced caspase-1 activity in control A549.pLex cells (Fig. 1 and 2) and it exerted an additive effect on the activation of caspase-1 induced by ORF9b (Fig. 1 and 2), which suggests that ORF9b and LPS+ATP may use distinct pathways to activate caspase-1. Of note, both long-term and transient expression of ORF3a, ORF7a, ORF8 and ORF10, as well as stable expression of ORF7b, inhibited the activation of caspase-1 induced by LPS+ATP in control A549.pLex cells (Fig. 1 and 2).

To examine if the observed inhibition of basal and LPS+ATP-induced caspase-1 activity by ORF3a, ORF7a, ORF8 and ORF10 affected the activation of caspase-1 by ORF9b, A549 cells stably integrating ORF9b (A549.ORF9b), which displays high caspase-1 activity relative to A549.pLex cells, were transiently transduced with lentiviral particles for the expression of each of the other accessory proteins or their control vector, pLex. It was observed that ORF3a and ORF6, but not ORF7a, ORF8 or ORF10, significantly attenuated the caspase-1 activity induced by ORF9b (Fig. 3). Interestingly, expression of ORF10 enhanced the caspase-1 activity induced by ORF9b (Fig. 3). Remarkably, transient transduction of any of the other accessory proteins inhibited the caspase-1 activity of A549.ORF9b cells (Fig. 3), to a greater extent particularly by ORF3a and ORF7b, but also ORF8 and ORF10 (Fig. 3).

As all accessory proteins are concomitantly expressed upon infection by SARS-CoV-2, all the accessory proteins under study were simultaneously expressed in parental A549 cells (A549wt), treated, or not, with LPS+ATP after transient transduction of ORFs (48 h). In parallel, stable A549.OR9b cells were subjected to transient co-transductions with lentiviruses for expression of the rest of accessory proteins. It should be noted that, in these experiments, each of the 10 accessory proteins is expressed at roughly equimolar levels, i.e., about 1/10 of the expression levels per cell relative to experiments with expression of individual accessory proteins. Concomitant expression of all accessory proteins in A549wt cells strongly (≈ 10-fold) induced caspase-1 activity, while it did not significantly affect the high levels of caspase-1 activity of A549.ORF9b cells (Fig. 4A). Likewise, transient expression of all accessory proteins in A549wt cells, including ORF9b (lane "all ORFs"), did not hinder the induction of caspase-1 activity by LPS+ATP (Fig. 4A), although the induction was attenuated as compared to the expression of ORF9b alone (compare to Fig. 1B, LPS+ATP, lanes "pLex" and "ORF9b"). Moreover, transient expression of all ORFs (except ORF9b) in stable A549.ORF9b cells abrogated the LPS+ATP-induced additive caspase-1 activation (Fig. 4A). These observations suggest that, while some SARS-CoV-2 accessory proteins, notably ORF3a, ORF7b and ORF10, counter inflammasome activation pathways triggered by LPS+ATP, they do not overcome the induction of caspase-1 activity by ORF9b, when expressed at levels likely paralleling those expected from SARS-CoV-2 infection, in which all accessory proteins are presumably expressed simultaneously. In turn, these results suggest that ORF9b and LPS+ATP activate caspase-1 through distinct pathways. The induction of caspase-1 activation by SARS-CoV-2 accessory proteins is abolished by the NLRP3 inflammasome inhibitor, MCC950, both without and with LPS+ATP (Fig. 4B), indicating that SARS-CoV-2 ORF9b induces caspase-1 activation through the NLRP3 inflammasome. Furthermore, these results suggest that the signals from ORF9b and LPS+ATP likely act divergently upstream of the NLRP3 inflammasome.

### Virtual screening to identify ORF9b dimerization inhibitor compounds.

The strong induction of inflammasome-dependent caspase-1 activity by ORF9b led us to hypothesize that this accessory protein might be a major driver of the unbridled cytokine release and inflammation associated with severe COVID-19. This prompted the development of small molecule inhibitors targeting ORF9b, potentially capable of mitigating the excessive activation of the inflammasome and consequent cytokine storm induced by SARS-CoV-2.

ORF9b can occur as monomers or homodimers (Ayinde et al., 2022) and, while monomers interact with TOM70 (Gordon et al., 2020a; Jiang et al., 2020; Gao et al., 2021; Ayinde et al., 2022; Thorne et al., 2022; Jin et al., 2023), in competition with HSP90 (Brandherm et al., 2021; Gao et al., 2021; Ayinde et al., 2022), ORF9b homodimers have been reported to directly associate with membranes (Meier et al., 2006; Jin et al., 2023). After modeling of a solved structure of the ORF9b homodimer (PDB:6Z4U), a blind docking screening was conducted on the entire homodimerization surfaces of the proteins, using as ligand sources the Diversity Library - European Chemical Biology Library (https://www.eu-openscreen.eu/services/compound-collection/european-chemical-biology-library-ecbl-diversity-library.html), with 96,096 diverse compounds, the Fragment Library - Fragment-Based lead discovery, with 1,056 fragments from the ECBL library (https://www.eu-openscreen.eu/services/compound-collection/fragment-library-fbld.html) and the SciFinder database of small compound structures (https://www.cas.org/solutions/cas-scifinder-discovery-platform/cas-scifinder-n). The dimeric ORF9b structure was constructed with 29 hydrogen bonds between two protomers and two salt bridges formed by residue R58 of one protomer and residue E90 of the other protomer (Jin et al., 2023).

Following an iterative process of molecular dynamics, docking and energy scoring, a total of 10 ligands were selected based on their free binding affinity energies on the ORF9b protein and position of the ligand binding sites on the homodimerization surface of the homodimer complex. The selected ligands were also predicted to interact with favorable energies with the ORF9b monomer, on a surface involved both in homodimerization and in binding to TOM70 (Gao et al., 2021). As such, the use by ORF9b of the same surface for the formation of homodimers and also for interaction with TOM70 implies that these are mutually exclusive interactions (Gao et al., 2021) and, as a corollary, that compounds binding to this surface may potentially prevent both the formation of ORF9b homodimers and its interaction with TOM70.

### An ORF9b dimerization inhibitor abrogates caspase-1 activation and causes mitochondrial eviction of ORF9b.

Next, the ability compounds selected by virtual screening as potential ORF9b ligands, was tested for their ability to prevent homotypic ORF9b interactions, as determined by surface plasmon resonance. For this, recombinant GST-ORF9b was immobilized on CM5 chips and free GST-ORF9b protein, with or without pre-incubation with 5 µM of each of the compounds, allowed to interact in the mobile phase with the GST-ORF9b-derivatized chips. As a control, GST was used in the mobile phase in place of chimeric GST-ORF9b. Binding of GST-ORF9b to immobilized GST-ORF9b was significantly inhibited by one of the compounds, hereafter designated Compound 1 (Fig. 5A).

The major interactions of Compound 1 on ORF9b are residues L52, K97 (hydrogen bonds), V15, L46, P53, L54, V76, 78 and V96 (hydrophobic interactions). Several such residues are critical for the homodimerizacion of ORF9b, notably P51, S53, V96 and K97 (Jin et al., 2023). Also of note, L46, L52 and S53 participate in the interaction of ORF9b with TOM70 (Gao et al., 2021). As such, these interactions both mechanistically explain the inhibition of ORF9b homodimerization by Compound 1 and predict that this compound and/or its derivatives are also likely to disrupt the interaction of ORF9b monomers with TOM70.

It was shown above that ORF9b is a strong inducer of caspase-1 activation. As such, the ability of Compound 1 to affect this function was tested. Indeed, this compound significantly inhibited the activation of caspase-1 in A549.ORF9b and THP-1. ORF9b cells at concentrations in the nanomolar range (Fig. 6 and 7). In A549.ORF9b cells, Compound 1 caused a rapid and remarkable partial eviction of ORF9b from mitochondria and relocalization to cytoplasmic structures (Fig. 8), in contrast to the exclusively mitochondrial localization of ORF9b in untreated cells. Collectively, these observations indicate that Compound 1 4240 inhibits the dimerization of ORF9b, thereby preventing its mitochondrial localization and activation of inflammasome-mediated caspase-1.

### Compound 1 derivatives as ORF9b inhibitors

Given the strong activity of Compound 1 on ORF9b homotypic interaction, ORF9b-induced caspase-1 activation and mitochondrial localization, the chemical space was explored to identify further ligands with similar structural features that might provide with structural diversity which may aid in subsequent chemical optimization. To this end, a round of constrained docking was conducted specifically overlapping with Compound 1 in the Compound 1-ORF9b complex model, using the SciFinder database as a source of ligands. Iterations of selection on the basis of free binding energies, classical molecular dynamics simulations and binding energy rescoring, yielded five Compound 1-similar ligands, designated Compounds 2 through 5 (Fig. 5 and Table 1).

**Table 1. Free-binding energies and residence times from molecular dynamics of Compound 1 and derivative compounds on the ORF9b homodimerization surface.**

| **LIGAND** | **ΔG_{AVE} (10 ns) (KCAL/MOL)** | **ΔG_{AVE} (RT) (kcal/mol)** | **Residence Time (ns)** |
|---|---|---|---|
| **Compound 1** | -29,00 | -27,95 | 60 |
| **Compound 2** | -31,95 | -32,34 | 80 |
| **Compound 4** | -26,12 | -25,08 | 200 |
| **Compound 5** | -21,30 | -23,15 | 120 |

These structures served as guides for *de novo* synthesis of the corresponding compounds, as well as additional compounds with small modifications and synthetic intermediate compounds (designated compounds 6-9). Eight of these nine novel compounds were analyzed for their ability to inhibit ORF9b-induced activation of caspase-1 and cytokine release, and their ability to cause mitochondrial eviction of ORF9b. Similar to Compound 1, Compounds 2, 4, 5, 6 and 7 inhibited ORF9b-induced caspase-1 activation in A549 (Fig. 9A) and THP-1 (Fig. 9B) cells. Compounds 1, 2 and 6 inhibited the ORF9b-induced release of IL-1β, IL-18 and IL-10, but not TNF-α, in THP-1 cells (Fig. 10). As a control, the NLRP3 inflammasome inhibitor MCC950 abrogated the release of all these cytokines, including TNF-α. Similar to Compound 1, Compounds 2 and 6 were also capable of causing mitochondrial eviction of ORF9b in A549.ORF9b cells at low micromolar concentrations (Fig. 11). Compounds 3 and 8 exhibited much lower or no ORF9b-induced caspase-1 inhibitory activity in A549 or THP-1 cells and did not cause mitochondrial eviction of ORF9b in A549.ORF9b cells (Fig. 11).

### Compound 1 and derivatives restore IFN-I responses compromised by ORF9b.

ORF9b is one of several SARS-CoV-2 proteins that interfere with type I and III interferon signaling through various mechanisms (Lei et al., 2020; Xia et al., 2020; Han et al., 2021; Rashid et al., 2022). The effect of SARS-CoV-2 ORF9b expression on IFN-β induced responses in A549 lung carcinoma cells and THP-1 monocyte-macrophage cells was tested, and the impact of our ORF9b dimerization inhibitors on this response. Exposure to IFN-β of A549.ORF9b or THP-1. ORF9b cells strongly stimulated the expression of the IFN-I/III stimulated genes OAS3 and ISG15 in control cells (Fig. 7A, B). This response was significantly mitigated in cells expressing ORF9b Fig. 7A, B), and was largely restored by ORF9b inhibitors Compound 1, Compound 2 and Compound 6, but not by the structural analog Compound 3, which also fails to inhibit the induction of caspase-1 activity by ORF9b.

## Claims

1. A compounds of formula **I:** 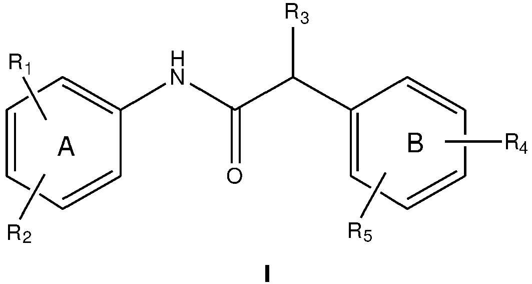 or a pharmaceutical salt thereof, wherein:
R₁ represents -H, -CN, -CF₃, -CHO, -CH₂OH, -F, -Cl, -Br, -I, -NH₂ or -(C₁-C₃)-alkyl;
R₂ represents -NH₂, -NO₂, -NH-OH, -NO, -NH-NH₂, -NH-COCH₃, -NH-COH, -NH-CO- -OEt, -NH-CO-OMe or -NH-CO-NH₂;
R₃ represents -H, -OH or -(C₁-C₃)-alkyl, or, alternatively, R₃ represents a chain selected from -CH=CH-CH=, -CH₂-O-, -CH₂-NH, -CH₂-S, -CH=CH-N=, =CH-O-, =CH-NH- and =CH-S- which is attached to the phenyl ring (B) at C-2 position;
each R₄ and R₅ independently represents -H, -F, -Cl, -Br, -I, -NO₂, -CF₃, -OCH₃, - OCF₃, -SCH₃, -(C₁-C₃)-alkyl, -CN, -COCH₃, -COH, -OH, -NH₂, -COOMe or -COOEt;
phenyl ring (A) is optionally fused to Cy, at any available position;
phenyl ring (B) is optionally fused to Cy₂ at any available position;
each of Cy, and Cy₂ independently represents a 5- or 6-membered carbocyclic ring that can be saturated, partially unsaturated or aromatic, and which can optionally contain 1 or 2 heteroatoms selected from N, S and O, wherein said ring can be bonded to the rest of the molecule through any available C or N atom at any available position, and which can be optionally substituted by 1 or 2 R₆ groups at any available position; and
each R₆ independently represents -H, -F, -Cl, -Br, -I, -NO₂, -CF₃, -OCH₃, -OCF₃, - SCH₃, -(C₁-C₃)-alkyl, -CN, -COCH₃, -COH, -OH, -NH₂, -COOMe or -COOEt.

2. The compound of formula **I** according to claim 1, wherein the compound of formula **I** is a compound of formula **Ia:** 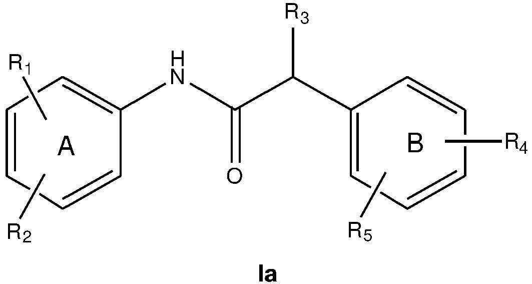 or a pharmaceutical salt thereof, wherein:
R₁ represents -H, -CN, -CF₃, -CHO, -CH₂OH, -F, -Cl, -Br, -I or -(C₁-C₃)-alkyl;
R₂ represents -NH₂, -NO₂, -NH-OH, -NO, -NH-NH₂, -NH-COCH₃, -NH-COH, -NH-CO- -OEt, -NH-CO-OMe or -NH-CO-NH₂;
R₃ represents -H, -OH or -(C₁-C₃)-alkyl, or, alternatively, R₃ represents a chain selected from -CH=CH-CH=, -CH₂-O-, -CH₂-NH, -CH₂-S, -CH=CH-N=, =CH-O-, =CH-NH- and =CH-S- which is attached to the phenyl ring (B) at C-2 position; and
each R₄ and R₅ independently represents -H, -F, -Cl, -Br, -I, -NO₂, -CF₃, -OCH₃, - OCF₃, -SCH₃, -(C₁-C₃)-alkyl, -CN, -COCH₃, -COH, -OH, -NH₂, -COOMe or -COOEt.

3. The compound of formula **I** according to any of claims 1 or 2, wherein R₁ represents -H, -CH₃, -F, -Cl, -Br or -I.

4. The compound of formula **I** according to any of claims 1 to 3, wherein R₂ represents -NH₂ or -NO₂.

5. The compound of formula **I** according to any of claims 1 to 4, wherein R₃ represents -H, -OH or-CH₃, or, alternatively, R₃ represents a CH=CH-CH= chain which is attached to the phenyl ring (B) at C-2 position.

6. The compound of formula **I** according to any of claims 1 to 5, wherein each R₄ and R₅ independently represents -H, -F, -Cl, -Br, -I or-OCH₃.

7. The compound of formula **I** according to any of claims 1 to 6, wherein:
R₄ represents -H; and
R₅ represents -H, -F, -CI or -OCH₃.

8. The compound of formula **I** according to claim 1, wherein the compound of formula **I** is selected from:

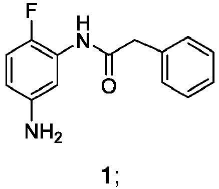

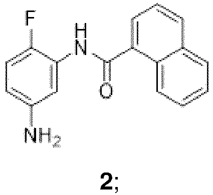

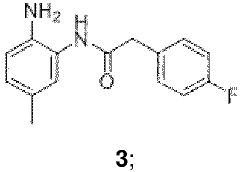

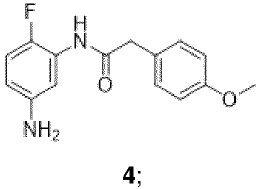

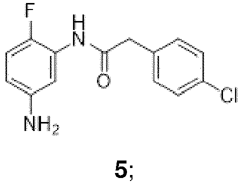

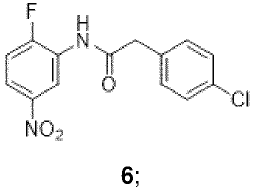

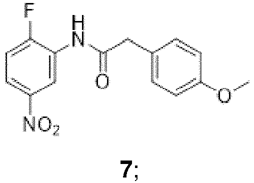

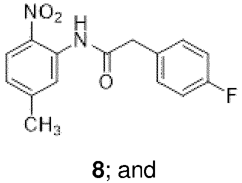

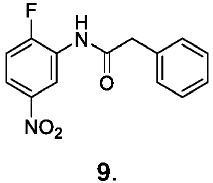

9. A pharmaceutical composition which comprises the compound of formula **I** according to any of claims 1 to 8 or a pharmaceutically acceptable salt thereof and one or more pharmaceutically acceptable excipients.

10. A compound of formula **I** according to any of claims 1 to 8 or a pharmaceutically acceptable salt therefore for use as a medicament.

11. A compound of formula **I** according to any of claims 1 to 8 or a pharmaceutically acceptable salt thereof for use in the treatment of hyperinflammation induced by viral infection.

12. The compound of formula **I** or a pharmaceutically acceptable salt thereof for the use according to claim 11, wherein viral infection is induced by highly pathogenic coronaviruses.

13. The compound of formula **I** or a pharmaceutically acceptable salt thereof for the use according to claim 12, wherein the highly pathogenic coronavirus is SARS-CoV-2.
